## Europäisches Patentamt

## European Patent Office

## Office européen des brevets

(11) Veröffentlichungsnummer: **0 183 991**
**B1**

(12) # EUROPÄISCHE PATENTSCHRIFT

(45) Veröffentlichungstag der Patentschrift:
**14.06.89**

(51) Int. Cl.⁴: **G 01 N 33/50, C 12 Q 1/00**

(21) Anmeldenummer: **85113860.2**

(22) Anmeldetag: **31.10.85**

(54) Verfahren zur Vollblutauftrennung.

(30) Priorität: **10.11.84 DE 3441149**

(43) Veröffentlichungstag der Anmeldung:
**11.06.86 Patentblatt 86/24**

(45) Bekanntmachung des Hinweises auf die Patenterteilung:
**14.06.89 Patentblatt 89/24**

(84) Benannte Vertragsstaaten:
**BE CH DE FR GB IT LI NL SE**

(56) Entgegenhaltungen:
**EP-A- 0 045 476**
**EP-A- 0 057 110**
**EP-A- 0 143 574**
**DE-A- 458 793**
**DE-A- 1 498 577**
**DE-A- 2 038 722**

(73) Patentinhaber: **MERCK PATENT GESELLSCHAFT MIT BESCHRÄNKTER HAFTUNG, Frankfurter Strasse 250 Postfach 4119, D-6100 Darmstadt (DE)**

(72) Erfinder: **Limbach, Berthold, Dr., Otto Hahn-Strasse 8, D-6104 Seeheim-JH 3 (DE)**
Erfinder: **Helger, Roland, Dr., Ludwigshöhstrasse 85, D-6100 Darmstadt (DE)**

## Beschreibung

Die Erfindung betrifft ein Verfahren zur einfachen Abtrennung von Plasma oder Serum von Vollblut bei gleichzeitiger Verdünnung durch ein Verdünnungsmittel.

Die quantitative Analyse der im Blut vorhandenen biologischen Substanzen ist für die klinische Chemie von entscheidender Bedeutung. Im Gegensatz zur Analyse von anderen Körperflüssigkeiten, wie Urin, ist eine direkte Bestimmung dieser Substanzen im Blut jedoch nur in bestimmten Fällen möglich. Üblicherweise müssen vor der eigentlichen Analyse die störenden Bestandteile, vornehmlich die Erythrozyten, abgetrennt werden. Dabei muss besonders darauf geachtet werden, dass keine Beeinflussung des Analysenresultates durch Hämolyse eintritt. Das am meisten verwendete Trennverfahren ist die Zentrifugation der zu analysierenden Blutproben. Als nachteilig erweist sich dabei jedoch der apparative Aufwand sowie die erforderlichen grossen Probevolumina. Ausserdem sind für die Druchführung qualifiziertes Personal sowie umfangreiche Sicherheitsvorkehrungen für die Handhabung und den Transport der Serenproben erforderlich. Darüber hinaus wirft die Trennung von Probenvorbereitung und eigentlicher Analyse Probleme mit der Probenidentifikation auf.

Aus dem Stand der Technik sind Verfahren zur Vollblutauftrennung bekannt, die auf der Agglutination der Erythrozyten durch Zusatz von Lectinen beruhen. Dabei wird die Vollblutprobe mit den entsprechenden Substanzen in einem geeigneten Gefäss versetzt und nach einer bestimmten Zeit das Serum oder Plasma entnommen. Das Gefäss ist dabei im einfachsten Fall das Blutentnahmerohr selbst (DE-A-2 038 722) oder es hat die Form eines Objektträgers (DE-A-1 498 577) oder eine andere beliebige Form. Die Lectine sind entweder als Feststoff in die entsprechenden Gefässe eingebracht oder an in den Gefässen enthaltenen Materialien immobilisiert (EP-A-57 110). Allen diesen Systemen ist gemeinsam, dass das vom Vollblut abgetrennte Serum oder Plasma nach einer bestimmten Zeit aus dem Auftrennbereich entfernt wird, um in einem anderen Bereich der weiteren Analyse zugeführt zu werden. Dabei bleiben die abgetrennten Erythrozyten sowie ein Teil des aufgetrennten Serums oder Plasmas in der Auftrennzone zurück. Diese nicht vollständige Überführung des gebildeten Serums oder Plasmas erfordert für die weitere Analyse entweder ein Abmessen der einzusetzenden Probemenge im Verhältnis zur Gesamtreaktionslösung oder aber ein Arbeiten ohne Verdünnen der Probelösung. Diese Verfahren weisen demnach ebenfalls eine Reihe von Nachteilen auf.

Der vorliegenden Erfindung liegt die Aufgabe zugrunde, ein Verfahren zur Vollblutauftrennung zur Verfügung zu stellen, das einerseits einfach und problemlos durchzuführen ist und andererseits auf eine einfache Weise mit der eigentlichen Bestimmungsreaktion gekoppelt und in ein gemeinsames Analysensystem integriert werden kann.

Überraschenderweise wurde gefunden, dass auf der Basis einer mit Lectin imprägnierten Matrix ein Verfahren zur Vollblutauftrennung konstruiert werden kann, bei dem die Auftrennzone nach Aufgabe der Vollblutprobe mit einem Verdünnungspuffer mehrmals durchspült werden kann, wobei die zu bestimmende Substanz reproduzierbar ausgespült wird, ohne dass eine die Analyse störende Hämolyse auftritt.

Besonders überraschend ist dabei, dass die zur Abtennung eingesetzten Lectine nicht an die Matrix immobilisiert werden müssen, wie in dem in der EP-OS 57 110 beschriebenen Verfahren, und die Abtrennreaktion weniger als 30 Sekunden erfordert gegenüber 0,5 bis 3 Stunden bei dem in der DE-OS 2 038 722 beschriebenen Verfahren.

Gelöst wird die Aufgabe dadurch, dass die aufzutrennende Vollblutprobe auf eine mit einem Lectin bzw. einem Lectingemisch imprägnierte Matrixschicht mit speziellen Matrixeigenschaften aufgegeben wird und anschliessend die mit Vollblut getränkte Matrixschicht mit einem Verdünnungsmittel mehrfach durchspült wird.

Gegenstand der Erfindung ist ein Verfahren zur Vollblutauftrennung durch Aufgeben einer Vollblutprobe auf eine mit Lectin imprägnierte Matrix und Abtrennung des Plasmas oder Serums, das dadurch gekennzeichnet ist, dass die aus einem saugfähigen Material mit einem relativen Strömungswiderstand (prozentuale Erhöhung der Durchflusszeit einer Lösung durch eine Säule bei zusätzlicher Durchströmung der Matrix) von bis zu 40% bestehende und mit der Vollblutprobe getränkte Matrix mit einem Verdünnungsmittel aus einer isotonischen Pufferlösung mit einem pH-Wert im Bereich von 5,0 bis 10,5 mehrfach durchspült wird. Die Matrix wird vorzugsweise mehrfach gegenläufig durchspült.

Als Matrices können im erfindungsgemässen Verfahren alle saufgähigen Materialien mit Gewebe- oder Faserstruktur verwendet werden, die einen möglichst geringen relativen Strömungswiderstand besitzen. Als relativer Strömungswiderstand soll dabei der Widerstand verstanden werden, den ein bestimmtes Material einer durchströmenden Lösung entgegensetzt. Er wird aus der prozentualen Erhöhung der Durchflusszeit einer Lösung durch eine Säule bei zusätzlicher Durchströmung der Matrix ermittelt. Im allgemeinen wird bei Werten von < 30% keine, bei Werten von < 40% eine starke Störung von klinisch-chemischen Testverfahren erhalten. Eine besondere Bedeutung kommt dabei auch dem Saugvermögen des verwendeten Materials zu; das Material besitzt z.B. dann ein genügend grosses Saugvermögen, wenn eine innerhalb der maximal aufsaugbaren Flüssigkeitsmenge liegendes Flüssigkeitsvolumen in maximal 10 bis 15 Sekunden vollständig aufgesaugt wird. Als bevorzugte Matrix-Materialien haben sich Materialien aus Baumvoll- und Viskosegewebe sowie diese Materialien enthaltende Mischgewebe und Zellulosematerialien erwiesen. Die Materialien können z.B. im Falle von Baumwolle eine Fadendichte von 10–25 (Kette) und 10–20 (Schuss) Fäden/cm bei einem Flächengewicht von 100–400 g/m² haben.

Die im erfindungsgemässen Verfahren verwendete Matrix kann aus mehreren Lagen der genannten Materialien bestehen. Dabei kann die Anzahl der zur Auftrennung eingesetzten Matrixlagen stark variieren; sie ist einerseits abhängig von der Saug- und Trennkapazität des Gewebes und von dem Volumen der aufzutrennenden Blutprobe, andererseits limitiert die Erhöhung des Strömungswiderstands die Anzahl der Lagen. Im allgemeinen sind zur Vollblutauftrennung nach dem vorliegenden Verfahren 1 bis 4 Lagen ausreichend; bei besonders dünnen Materiallagen können gegebenenfalls auch mehr Lagen eingesetzt werden. Bei der Verwendung mehrerer Lagen kann die der Probenaufgabe am weitesten entfernte Lage zusätzlich hydrophobiert sein. Die Hydrophobierung erfolgt z.B. durch Aufgabe einer bei Raumtemperatur mit Paraffin gesättigten Lösung von Dichlormethan auf eine Matrixscheibe und anschliessendem Trocknen.

Als Lectine (Agglutinine) können alle Substanzen eingesetzt werden, die eine starke Agglutination der Erythrozyten bewirken. Geeignete Lectine sind z.B. solche aus Solanum tuberosum, Tritium vulgaris, Ricinus communis, Sojabohnen, Phaseolus vulgaris, Lentil, Weizenkeimen, Phytolacca americana. Ein Zusatz von die Agglutination verstärkender Substanzen, z.B. kationische Polymere, wie Hexadimethrinbromid, ein Polymer aus N,N,N',N'-Tetramethylhexamethylendiamin und Trimethylenbromid; N,N,N',N'-Tetramethyl-1,6-hexandiamin-Polymer mit 1,3-Dibrompropan; Poly(N,N,N',N'-Tetramethyl-N-trimethylenhexamethylendiammoniumdibromid) ist ebenfalls möglich. Als besonders geeignet haben sich Gemische von Lectinen mit einem geringen Anteil eines kationischen Polymeren erwiesen, z.B. Lectingemische von Sojabohnen und Solanum tuberosum im Verhältnis 2:1 mit 2% kationischem Polymer oder ein Lectingemisch aus Weizenkeimen, Sojabohnen, Phaseolus vulgaris im Verhältnis 1:2:8 mit ebenfalls 2% kationischem Polymer.

Das mehrfache Durchspülen der mit Vollblut getränkten Auftrennschicht ermöglicht eine photometrische Analyse der für die klinische Diagnostik wichtigen Parameter. Dabei sind aufgrund der Verdünnung auch die besonders störungsanfälligen sogenannten Endpunktverfahren für die in hoher Konzentration im Vollblut enthaltenden Substrate möglich. Das erfindungsgemässe Verfahren erlaubt es, einerseits durch das mehrfache Durchströmen der Aufbereitungszone mit dem Verdünnungsmittel eine genügend reproduzierbare Ausspülung der zu bestimmenden Substanzen zu erreichen, so dass eine weitere Pipettierung nicht erforderlich ist, andererseits durch die Wahl der restlichen Verfahrensparameter eine Störung der Analysenlösung durch Produkte der Vollblutauftrennung zu verhindern, ohne dass dadurch komplizierte Vorbereitungsschritte, z.B. das Immobilisieren der Lectine oder grössere Reaktionszeiten in Kauf genommen werden müssen. Der Vorgang des mehrfachen Durchspülens kann in beliebiger Form durchgeführt werden; vozugsweise in gegenläufiger Ausführungsform, bei der das Verdünnungsmittel abwechselnd die Matrix von der Seite der Probenaufgabe und dann von der der Probenaufgabe abgewandten Seite aus durchspült wird, da dies mit dem geringsten apparativen Aufwand durchgeführt werden kann. Dieser Vorgang ist im einfachsten Fall mit einer in eine Küvette eingetauchten Einwegspritze ausführbar. Selbstverständlich sind auch andere gegenläufige und nicht gegenläufige Ausführungsformen möglich sowie die Verbindung von Vollblutauftrennung und einer anschliessenden klinisch-chemischen Analyse in einem Gesamtreaktionsteil.

Als Vollblutprobe kann sowohl kapillares als auch venöses Blut eingesetzt werden. Die Probe kann entweder sofort nach Gewinnung oder bei Zugabe von gerinnungsverhindernden Soffen wie EDTA oder Citrat erst später auf den Auftrennbereich aufgegeben werden.

Als Verdünnungsmittel können isotonische Pufferlösungen eingesetzt werden, wobei alle gängigen Puffersubstsanzen, wie etwa Phosphat, Glycin, Citrat, Tris, Triethanolamin, PIPES usw., verwendet werden können. Der pH-Wert der eingesetzten Pufferlösung kann über einen Bereich von pH 5,0 bis 10,5 gewählt werden, bevorzugt sind Phosphat-, Triethanolamin- und Glycinpuffer im pH-Bereich von 6,5 bis 10.

Prinzipiell sind alle in der klinischen Chemie üblichen Substratbestimmungen nach Auftrennung der Vollblutprobe nach dem erfindungsgemässen Verfahren durchführbar. Durch die freie Wahl des Verdünnungsverhältnisses von Probe zu Gesamtlösung können ohne Schwierigkeiten sowohl die im Blut in hoher Konzentration vorkommenden Substrate als auch die geringen Aktivitäten von Proteinen bestimmt werden.

Die Erfindung wird durch die folgenden Beispiele näher erläutert.

Beispiel 1
Bestimmung des relativen Strömungswiderstandes

Jeweils eine Scheibe (⌀ 10 mm) verschiedener Matrices wurde mit 50 µl einer Lösung von 0,03 mol/l Phosphatpuffer, 0,1 mol/l Natriumchlorid, 1% kationischem Polymer, 13 µg Lectin aus Weizenkeimen und 25 µg Lectin aus Sojabohnen getränkt und getrocknet. Die so imprägnierten Scheiben wurden mit 30 µl EDTA-Vollblut versetzt und in eine Säule (⌀ 10 mm) oberhalb der Ausgangsöffnung (⌀ 1 mm) eingespannt. Bei versperrter Ausflussöffnung wurden 20 ml einer isotonischen Natriumchloridlösung eingefüllt und nach Öffnen der Sperre die Zeit bis zum vollständigen Austritt des eingegebenen Flüssigkeitsvolumens ermittelt. Über den Vergleich mit der Ausflusszeit bei einer Säule ohne Matrix ergibt sich der relative Strömungswiderstand als prozentuale Erhöhung der Durchflusszeit.

Beispiel 2

Bestimmung der Vollblutauftrenneigenschaften verschiedener Matrices

Nach Beispiel 1 imprägnierte Matrixscheiben wurden in einer Einwegspritze (⌀ 10 mm) mit Hilfe einer in der Mitte offenen Abdeckscheibe eingespannt und mit 30 µl EDTA-Blut getränkt. Nach 30 Sekunden wurde die Spritzenspitze in eine Küvette mit 1 ml einer Lösung enthaltend 0,03 mol/l Phosphatpuffer, pH 7,25 und 0,1 mol/l Natriumchlorid eingetaucht. Die Lösung wurde durch Einsaugen und Ausdrücken mehrmals gegenläufig durch die Matrixscheibe gespült. Dabei wurde nach jedem Spülvorgang an Hand der bei 405 nm gemessenen Extinktion der Anteil der Hämolyse ermittelt und qualitativ beurteilt. Die erhaltenen Ergebnisse sind in der folgenden Tabelle zusammengestellt. Dabei bedeutet +++ sehr gut, ++ gut, + ausreichend, – mangelhaft, –– völlig unzureichend. Für eine gute Vollblutauftrennung sind demnach ein möglichst geringer Strömungswiderstand und eine gute Saugfähigkeit der Matrix erforderlich.

| Matrix Nr. | Material | Fadendichte (Fäden/cm) Kette | Schuss | Flächengewicht (g/m²) | Saugfähigkeit | Durchflusszeit (sec) | relativer Strömungswiderstand (%) | Vollblutauftrennung nach 1 Spülvorgängen | Vollblutauftrennung nach 3 Spülvorgängen |
|---|---|---|---|---|---|---|---|---|---|
| 1 | Baumwolle | 25,0 | 18,7 | 120 | + | 29 | 2 | +++ | +++ |
| 2 | Baumwolle | 17,9 | 11,3 | 123 | + | 31 | 9 | +++ | ++ |
| 3 | Baumwolle | 13,6 | 19,9 | 316 | + | 36 | 26 | ++ | + |
| 4 | 68% Baumwolle 32%Polyester | – | – | 55 | + | 35 | 24 | ++ | ++ |
| 5 | Polyester | 76,9 | 39,8 | 108 | + | 32 | 12 | ++ | + |
| 6 | 70% Polyester 30% Viskose | 12,3 | 11,8 | 224 | + | 31 | 10 | +++ | ++ |
| 7 | 56% Polyester 44% Wolle | 31,9 | 34 | 187 | + | 44 | 54 | – | – |
| 8 | Wolle | 25,9 | 22,8 | 113 | + | 41 | 43 | – | – |
| 9 | Wolle | 24,1 | 22,2 | 116 | – | 31 | 10 | – | – |
| 10 | Zellulose | – | – | – | – | 30 | 5 | +++ | +++ |

Beispiel 3

Eignung verschiedener Pufferlösungen als Verdünnungsmittel

In drei Versuchsreihen wurde jeweils eine Matrixscheibe aus Baumwollgewebe (⌀ 10 mm) durch Aufgabe von 100 µl einer bei Raumtemperatur mit Paraffin gesättigten Lösung von Dichlormethan und anschliessendem Trocknen hydrophobiert und eine weitere mit jeweils einem der in der nachstehenden Tabelle aufgeführten Lectinen imprägnierte Scheibe übereinander in eine Einwegspritze gelegt. Nach Aufgabe von 30 µl EDTA-Vollblut auf die so präparierte Auftrennzone wurde diese jeweils mit 1 ml Pufferlösung mit verschiedenen pH-Werten dreimal gegenläufig durchgespült. Über die Extinktion bei 405 nm wurden die Ergebnisse der Vollblutauftrennung qualitativ ermittelt. Wie die Tabelle zeigt, sind die als Verdünnungsmittel gewählten Pufferlösungen mit pH-Werten zwischen 5,5 und 10,5 sehr gut geeignet. Die Bewertungen entsprechen denen aus Beispiel 2.

pH-Wert der Pufferlösung

| Lecitin aus: | 1 5,5 | 1 6,0 | 1 6,5 | 1 7,0 | 1 7,5 | 2 8,0 | 2 8,5 | 2 9,0 | 2 9,5 | 3 10,0 | 3 10,5 | 3 11,0 |
|---|---|---|---|---|---|---|---|---|---|---|---|---|
| Ricinus communis Weizenkeimen + Phytolacca americana (2,5:1) | + | + | + + | + + | + + | + + | + + | + + | + + | + + | + | – |
| Weizenkeimen + Sojabohnen + Phaseolus vulgaris (1:2:8) | – | – | + | + + | + + | + + | + + | + + | + + | + + | + | – |
| | – | – | + | + + + | + + + | + + + | + + + | + + + | + + + | + + | + + | + | – |

1 = 0,03 mol/l Phospaht + 0,3 mol/l NaCl
2 = 0,03 mol/l Triethanolamin + 0,3 mol/l NaCl
3 = 0,03 mol/l Gycin + 0,3 mol/l NaCl

Beispiel 4
Bestimmung von Glucose im Vollblut

Eine Matrixscheibe (⌀ 10 mm) aus Baumwollgewebe (Matrix 1 aus Beispiel 2) wurde analog Beispiel 3 hydrophobiert, eine weitere wurde mit 50 µl einer Lösung enthaltend 0,01 mol/l Phosphatpuffer, pH 7,25, 2% kationisches Polymer, 16 µg Lectin aus Sojabohnen und 8 µg Lectin aus Solanum tuberosum getränkt und getrocknet. Beide Scheiben wurden übereinander (die hydrophobierte als untere) in eine Einwegspritze (⌀ 10 mm) gelegt. Auf die obere Scheibe wurden dann in verschiedenen Versuchen jeweils 20 µl einer EDTA-Vollblutprobe aufgegeben, die verschiedene Glucosekonzentrationen enthielt. Der Gehalt an Glucose wurde zusätzlich durch Bestimmung im Serum ermittelt. Anschliessend wurden die Vollblutauftrennzonen jeweils mit 1 ml einer Lösung aus 0,03 mol/l Phosphatpuffer, pH 7,25 und 0,3 mol/l Natriumchlorid in getrennten Versuchen einmal und dreimal gegenläufig durchspült und die Extinktion der erhaltenen Lösung bei 365 nm gemessen. Danach wurden 50 µl Reaktionslösung, enthaltend 100 kU/l Glucosedehydrogenase, 2 kU/l Mutarotase und 21 mmol/l NAD⁺ in 0,1 mol/l Phosphatpuffer, pH 7,6, zugegeben und nach 10 Minuten erneut die Extinktion der Gesamtlösung bei 365 nm gemessen. Aus der Extiktionsdifferenz erhält man durch Multiplikation mit dem Faktor F = 630 den Glucosegehalt der Ausgangsprobe; die entsprechenden Ergebnisse sind in der nachstehenden Tabelle aufgeführt. Insgesamt zeigt sich, dass bei mehrfachem gegenläufigen Durchspülen bessere Resultate erhalten werden als bei einfachem Durchspülen.

Glucosebestimmung

| im Serum [mg/dl] | nach dem erfindungsgemässem Verfahren | | | |
|---|---|---|---|---|
| | bei einfacher Durchspülung [mg/dl] | Standardabweichung von 6 Werten [%] | bei mehrfacher Durchspülung [mg/dl] | Standardabweichung von 6 Werten [%] |
| 59 | 52 | 21,0 | 61 | 2,4 |
| 78 | 86 | 12,9 | 74 | 3,0 |

| im Serum | nach dem erfindungsgemässem Verfahren | | | |
| --- | --- | --- | --- | --- |
| | bei einfacher Durchspülung | Standardab- weichung von 6 Werten | bei mehrfacher Durchspülung | Standardab- weichung von 6 Werten |
| [mg/dl] | [mg/dl] | [%] | [mg/dl] | [%] |
| 120 | 131 | 4,9 | 114 | 3,5 |
| 139 | 144 | 9,0 | 135 | 3,9 |
| 179 | 189 | 4,9 | 171 | 2,3 |
| 235 | 233 | 8,6 | 240 | 3,9 |
| 310 | 293 | 21,0 | 318 | 4,6 |
| 520 | 540 | 18,0 | 543 | 2,4 |

Beispiel 5
Bestimmung von Harnstoff im Vollblut

In eine Einwegspritze ($\varnothing$ 10 mm) wurde eine Matrixscheibe aus hydrophobiertem Baumwollgewebe (Matrix 1 aus Beispiel 2) und darüber eine weitere Matrixscheibe eingelegt, die mit einer Lösung aus 0,025 mol/l Phosphatpuffer, pH 8,0, 0,1 mol/l Natriumchlorid, enthaltend 2,5% kationisches Polymer sowie 19 µg Lectin aus Ricinus communis getränkt und dann getrocknet worden war. Auf die obere Scheibe wurden jeweils 15 µl einer EDTA-Vollblutprobe aufgegeben, die verschiedene Harnstoffkonzentrationen enthielt. Der Gehalt an Harnstoff wurde zusätzlich im Serum ermittelt. Anschliessend wurden die Vollblutauftrennzonen jeweils mit einer Lösung aus 0,03 mol/l Triethanolaminpuffer, pH 8,0 und 0,3 mol/l Natriumchlorid in getrennten Versuchen einmal und dreimal gegenläufig durchgespült. Danach wurden 50 µl Reaktionslösung enthaltend 25 kU/l Glutamat-Dehydrogenase, 0,2 mol/l Ketoglutarat, 4,6 mmol/l NADH und 30 mmol/l ADP in 0,3 mol/l Triethanolaminpuffer, pH 8,0, zugegeben und die Extinktion $E_1$ bei 334 nm nach 10 Minuten gemessen. Dann wurden 20 µl einer Lösung aus 450 kU/l Urease in 0,1 mol/l Triethanolaminpuffer, pH 8,0, zugegeben und nach einer weiteren Reaktionszeit von 10 Minuten bei Raumtemperatur die Extinktion $E_2$ bei 334 nm gemessen. Aus $E_1 - E_2$ wurde durch Multiplikation mit dem Faktor 165 der Harnstoffgehalt der Ausgangsprobe bestimmt; die entsprechenden Ergebnisse sind in der nachstehenden Tabelle aufgeführt. Es zeigt sich, dass insbesondere bei mehrfachem gegenläufigem Durchspülen sehr gute Ergebnisse erhalten werden.

Harnstoffbestimmung

| im Serum | nach dem erfindungsgemässem Verfahren | | | |
| --- | --- | --- | --- | --- |
| | bei einfacher Durchspülung | Standardab- weichung von 6 Werten | bei mehrfacher Durchspülung | Standardab- weichung von 6 Werten |
| [mg/dl] | [mg/dl] | [%] | [mg/dl] | [%] |
| 32 | 31 | 17,4 | 31 | 3,9 |
| 40 | 39 | 6,8 | 41 | 3,1 |
| 49 | 46 | 7,6 | 48 | 2,0 |
| 52 | 51 | 12,1 | 53 | 2,8 |
| 61 | 56 | 9,1 | 62 | 3,2 |
| 72 | 68 | 5,9 | 74 | 3,3 |
| 88 | 89 | 7,2 | 91 | 2,4 |
| 93 | 88 | 6,1 | 90 | 3,5 |
| 99 | 99 | 11,0 | 108 | 2,4 |
| 125 | 122 | 10,5 | 121 | 3,8 |

Beispiel 6
Bestimmung von $\gamma$-Glutamyltranspeptidase im Vollblut

In einer Einwegspritze wurden über einer Matrixscheibe von hydrophobiertem Baumwollgewebe (Matrix 1 aus Beispiel 2) vier Matrixscheiben aus Zellulosegewebe (Matrix 10 aus Bespiel 2) gelegt, die mit 100 µl einer Lösung von 0,015 mol/l Phosphatpuffer, pH 6,5, 0,3 mol/l Natriumchlorid enthaltend 2% kationisches Polymer, 25 µg Lectin aus Sojabohnen und 13 µg Lectin aus Weizenkeimen getränkt waren. Auf die so vorbereiteten Scheiben wurden jeweils 70 µl EDTA-Vollblutproben aufgegeben, die verschiedene $\gamma$-GT-Aktivitäten enthielten. Zur Kontrolle wurden Serumbestimmungen durchgeführt. Anschliessend wurden die Vollblutauftrennzonen jeweils mit 1 ml einer Lösung aus 0,015 mol/l Phosphatpuffer, pH 6,5 und 0,3 mol/l Natriumchlorid in getrennten Versuchen einmal und dreimal gegenläufig durchgespült. Zu beiden Versuchsreihen wurden je 100 µl einer Lösung aus 1 mol/l Glycylglycin in 0,3 mol/l Trispuffer, pH 8,25, zupipettiert und die Lösungen auf 25°C thermostatisiert. Nach vier Minuten erfolgte eine weitere Zugabe von 100 µl einer Lösung aus 30 mmol/l L-$\gamma$-Glutamyl-3-carboxy-4-nitroanilid in

0,3 mol/l Trispuffer, pH 8,25. Anschliessend wurde 7 Minuten lang die Extinktionszunahme bei 405 nm registriert und aus den ermittelten Extinktionsdifferenzen pro Minute durch Multiplikation mit dem Faktor F = 3 600 die Aktivität der Ausgangsprobe bestimmt. Wie die Ergebnisse in der nachstehenden Tabelle zeigen, liefert die Methode mit mehrfachem gegenläufigem Durchspülen bessere Resultate als mit einfachem Durchspülen.

$\gamma$-GT-Bestimmung

| im Serum | nach dem erfindungsgemässen Verfahren | | | |
| | bei einfacher Durchspülung | Standardabweichung von 6 Werten | bei mehrfacher Durchspülung | Standardabweichung von 6 Werten |
| [mg/dl] | [mg/dl] | [%] | [mg/dl] | [%] |
| --- | --- | --- | --- | --- |
| 13 | 14 | 24,0 | 14 | 8,9 |
| 19 | 18 | 13,0 | 18 | 5,7 |
| 31 | 30 | 16,0 | 30 | 4,8 |
| 56 | 56 | 7,5 | 52 | 4,3 |
| 66 | 63 | 15,0 | 70 | 0,8 |
| 84 | 84 | 13,0 | 85 | 4,5 |
| 87 | 88 | 9,3 | 92 | 4,9 |
| 106 | 108 | 6,2 | 105 | 3,3 |
| 115 | 112 | 7,1 | 116 | 4,1 |
| 140 | 139 | 6,0 | 138 | 4,7 |
| 215 | 209 | 8,1 | 195 | 5,2 |

## Patentansprüche

1. Verfahren zur Vollblutauftrennung durch Aufgeben einer Vollblutprobe auf eine mit Lectin imprägnierte Matrix und Abtrennung des Plasmas oder Serums, dadurch gekennzeichnet, dass die aus einem saugfähigen Material mit einem relativen Strömungswiderstand (prozentuale Erhöhung der Durchflusszeit einer Lösung durch eine Säule bei zusätzlicher Durchströmung der Matrix) von bis zu 40% bestehende und mit der Vollblutprobe getränkte Matrix mit einem Verdünnungsmittel aus einer isotonischen Pufferlösung mit einem pH-Wert im Bereich von 5,0 bis 10,5 mehrfach durchspült wird.

2. Verfahren nach Anspruch 1, dadurch gekennzeichnet, dass die Matrix mit dem Verdünnungsmittel mehrfach gegenläufig durchspült wird.

3. Verfahren nach den Ansprüchen 1 und 2, dadurch gekennzeichnet, dass als Matrix ein saugfähiges Material aus Baumwollgewebe, Baumwolle enthaltendem Mischgewebe, Zellulose, Viskose oder aus einem diese Bestandteile enthaltenden Material verwendet wird.

4. Verfahren nach den Ansprüchen 1 bis 3, dadurch gekennzeichnet, dass eine aus mehreren Lagen bestehende Matrix verwendet wird, wobei die der Probenaufnahme entfernteste Lage hydrophobiert sein kann.

## Revendications

1. Procédé pour la séparation du sang entier par application d'un échatillon de sang entier sur une matrice imprégnée de lectine et séparation du plasma ou du sérum, caractérisé en ce que la matrice constituée d'un matériau absorbant avec une résistance à l'écoulement relative (augmentation en pour cent du temps de passage d'une solution dans une colonne par un passage supplémentaire à travers la matrice) allant jusqu'à 40% et imprégnée avec l'échantillon de sang entier est traversée plusieurs fois par un diluant constitué d'une solution tampon isotonique avec une valeur de pH comprise dans l'intervalle de 5,0 à 10,5.

2. Procédé selon la revendication 1, caractérisé en ce que la matrice est traversée plusieurs fois en sens contraire par le diluant.

3. Procédé selon les revendications 1 et 2, caractérisé en ce que l'on utilise comme matrice un matériau absorbant en tissu de coton, en tissu mélangé contenant du coton, en cellulose, en viscose ou en un matériau contenant l'un de ces constituants.

4. Procédé selon les revendication 1 à 3, caractérisé en ce que l'on utilise une matrice constituée de plusieurs couches, la couche la plus éloignée de celle ayant reçu les échantillons pouvant être rendue hydrophobe.

## Claims

1. Method for the fractionation of whole blood by applying a sample of whole blood to a matrix impregnated with lectin and removal of the plasma or serum, characterised in that the matrix which is composed of an absorbent material having a relative flow resistance (percentage increase in the time taken by a solution to flow through a column when it additionally flows through the matrix) of up to 40% an which is impregnated with the sample of whole blood is flushed through several times with a diluent consisting of an isotonic buffer solution having a pH in the range 5.0 to 10.5.

2. Method according to Claim 1, characterised in that the matrix is flushed through with the diluent several times in opposite directions.

3. Method according to Claims 1 and 2, characterised in that the matrix used is an absorbent material of cotton fabric, cotton-containing blended fabric, cellulose, viscose or a material containing these constituents.

4. Method according to Claims 1 to 3, characterised in that a matrix composed of several layers is used, it being possible for the layer furthest from the absorption of the sample to be rendered hydrophobic.